# EUROPEAN PATENT APPLICATION

(11) **EP 4 817 548 A1**
(43) Date of publication of application: **07.10.2026**
(21) Application number: 26161734.4
(22) Date of filing: 02.03.2026
(51) Int. Cl.: A61B 34/20, A61B 90/00

(54) **MEDICAL SUPPORT DEVICE, MEDICAL SUPPORT METHOD, AND MEDICAL SUPPORT PROGRAM**

(30) Priority: 24.03.2025 JP 2025048278
(71) Applicant: FUJIFILM Corporation, Tokyo 106-8620 (JP)
(72) Inventor: MASUMOTO, Jun, Tokyo, 106-8620 (JP); SASUGA, Saeko, Tokyo, 106-8620 (JP)
(74) Representative: Dehns Germany Partnerschaft mbB

(57) **Abstract**

Provided are a medical support device (11), a medical support method, and a medical support program that can improve general-purpose properties in a case of estimating a position and an orientation of a medical instrument.

A medical support device (11) includes a processor (41), in which the processor (41) is configured to acquire, at a first time point (t1), a surgical field image (21) obtained by imaging, with a camera, a surgical field including a first medical instrument to which a marker is assigned, identify, based on the marker included in the surgical field image (21), first position and orientation information indicating a position and an orientation of the first medical instrument, the first position and orientation information being information at the first time point (t1), acquire second position and orientation information indicating a position and an orientation of a second medical instrument connected to the first medical instrument, the second position and orientation information being information at the first time point (t1) and a second time point (t2), derive a relative positional relationship between the first medical instrument and the second medical instrument based on the first position and orientation information and the second position and orientation information at the first time point (t1), and estimate the first position and orientation information at the second time point (t2) based on the relative positional relationship and the second position and orientation information at the second time point (t2).

## Description

### Technical Field

The present disclosure relates to a medical support device, a medical support method, and a medical support program.

### Background Art

In the related art, a technology of identifying a position and an orientation of a medical instrument and supporting medical care such as a surgery and an examination based on the identified position and orientation of the medical instrument is known.

For example, JP2024-131034A discloses deriving position and orientation information of a medical device within a captured image based on a marker included in the captured image, and displaying a composite image in which medical support information is superimposed at a position identified within the captured image based on the position and orientation information. In addition, for example, JP2021-146042A discloses acquiring relative positions of a diseased area and an operation tool attached to an arm of a surgical support robot from imaging results of markers attached to the diseased area and the operation tool, and displaying virtual images of the diseased area and the operation tool according to the relative positions and a patient's posture.

### SUMMARY

As in the related art, the position and the orientation of the medical instrument are identified based on a surgical field image including the medical instrument. However, during a surgery, the medical instrument is moved, so that the medical instrument is not always included in a field of view of the surgical field image, and it is sometimes difficult to identify the position and the orientation of the medical instrument.

The present disclosure provides a medical support device, a medical support method, and a medical support program that can improve general-purpose properties in a case of estimating a position and an orientation of a medical instrument.

According to a first aspect of the present disclosure, there is provided a medical support device comprising: a processor, in which the processor is configured to acquire, at a first time point, a surgical field image obtained by imaging, with a camera, a surgical field including a first medical instrument to which a marker is assigned, identify, based on the marker included in the surgical field image, first position and orientation information indicating a position and an orientation of the first medical instrument, the first position and orientation information being information at the first time point, acquire second position and orientation information indicating a position and an orientation of a second medical instrument connected to the first medical instrument, the second position and orientation information being information at the first time point and a second time point, derive a relative positional relationship between the first medical instrument and the second medical instrument based on the first position and orientation information and the second position and orientation information at the first time point, and estimate the first position and orientation information at the second time point based on the relative positional relationship and the second position and orientation information at the second time point.

The surgical field image may include the second medical instrument to which a marker is assigned, and the processor may be configured to identify the second position and orientation information at at least one of the first time point or the second time point based on the marker included in the surgical field image.

The surgical field image may include the second medical instrument, and the processor may be configured to identify the second position and orientation information at at least one of the first time point or the second time point based on a characteristic shape of the second medical instrument included in the surgical field image.

The processor may be configured to acquire the second position and orientation information at the first time point, acquire drive information of the second medical instrument, and identify the second position and orientation information at the second time point based on the second position and orientation information at the first time point and the drive information.

The processor may be configured to acquire the second position and orientation information at the first time point, the second position and orientation information being relative to a viewpoint of the surgical field image, acquire drive information including a movement amount of the second medical instrument from the first time point to the second time point, and identify the second position and orientation information at the second time point based on the second position and orientation information at the first time point and the drive information.

The processor may be configured to acquire variation information indicating an amount of variation in the relative positional relationship after the first time point, and correct the relative positional relationship based on the variation information.

The processor may be configured to acquire an auxiliary image obtained by imaging the surgical field with a camera from a direction different from a direction in which the surgical field image is captured, and identify the second position and orientation information at the first time point and the second time point based on a feature portion of the second medical instrument included in the auxiliary image.

The auxiliary image may be captured to track the second medical instrument.

The processor may be configured to acquire parallax information of the surgical field image and the auxiliary image, and estimate the first position and orientation information at the second time point relative to a viewpoint of the surgical field image based on the relative positional relationship, the second position and orientation information at the second time point identified based on the auxiliary image, and the parallax information.

The first medical instrument may be an ultrasound probe that captures an ultrasound image, and the processor may be configured to select the surgical field image or the auxiliary image based on the first position and orientation information, and perform control of superimposing and displaying the ultrasound image on the selected image.

The processor may be configured to determine whether or not an amount of variation between the relative positional relationship at the first time point and the relative positional relationship at the second time point is within a predetermined threshold value, and, in a case where the amount of variation is within the threshold value, estimate the first position and orientation information at the second time point based on the relative positional relationship and the second position and orientation information at the second time point.

The processor may be configured to perform a first determination of whether or not the second medical instrument is connected to the first medical instrument and a second determination of whether or not the marker and a feature portion of the second medical instrument are included in the surgical field image, and set a time point at which both the first determination and the second determination are affirmative, as the first time point.

The processor may be configured to perform the first determination based on the surgical field image.

The processor may be configured to acquire drive information of the second medical instrument, and perform the first determination based on the drive information.

The processor may be configured to perform the first determination and the second determination over time, and, in a case where the second determination is negative, set a time point at which both the immediately preceding first determination and second determination are affirmative, as the first time point.

The processor may be configured to, after the first time point, restrict a drive amount of the second medical instrument such that an amount of variation in the relative positional relationship over time is within a predetermined threshold value.

The processor may be configured to determine whether or not the second medical instrument is connected to the first medical instrument, and implement the restriction in a case where the determination is affirmative.

The processor may be configured to receive designation as to whether or not to allow variation of the relative positional relationship after the first time point, and implement the restriction in a case where it is designated that the variation of the relative positional relationship is not allowed.

According to a second aspect of the present disclosure, there is provided a medical support method executed by a computer, the medical support method comprising: acquiring, at a first time point, a surgical field image obtained by imaging, with a camera, a surgical field including a first medical instrument to which a marker is assigned; identifying, based on the marker included in the surgical field image, first position and orientation information indicating a position and an orientation of the first medical instrument, the first position and orientation information being information at the first time point; acquiring second position and orientation information indicating a position and an orientation of a second medical instrument connected to the first medical instrument, the second position and orientation information being information at the first time point and a second time point; deriving a relative positional relationship between the first medical instrument and the second medical instrument based on the first position and orientation information and the second position and orientation information at the first time point; and estimating the first position and orientation information at the second time point based on the relative positional relationship and the second position and orientation information at the second time point.

According to a third aspect of the present disclosure, there is provided a medical support program for causing a computer to execute: acquiring, at a first time point, a surgical field image obtained by imaging, with a camera, a surgical field including a first medical instrument to which a marker is assigned; identifying, based on the marker included in the surgical field image, first position and orientation information indicating a position and an orientation of the first medical instrument, the first position and orientation information being information at the first time point; acquiring second position and orientation information indicating a position and an orientation of a second medical instrument connected to the first medical instrument, the second position and orientation information being information at the first time point and a second time point; deriving a relative positional relationship between the first medical instrument and the second medical instrument based on the first position and orientation information and the second position and orientation information at the first time point; and estimating the first position and orientation information at the second time point based on the relative positional relationship and the second position and orientation information at the second time point.

According to the aspects, the medical support device, the medical support method, and the medical support program of the present disclosure can improve general-purpose properties in a case of estimating a position and an orientation of a medical instrument.

### BRIEF DESCRIPTION OF DRAWINGS

FIG. 1 is a diagram showing a state of an inside of a body in a robotic surgery.
FIG. 2 is a diagram showing an example of a hardware configuration of a medical support device.
FIG. 3 is a diagram showing an example of a functional configuration of the medical support device according to a first embodiment.
FIG. 4 is a diagram showing a relationship in a position and an orientation between a marker and an ultrasound probe.
FIG. 5 is a diagram showing an example of a surgical field image, an ultrasound image, and a superimposed image.
FIG. 6 is a diagram showing a relationship in a position and an orientation between a marker and an ultrasound probe.
FIG. 7 is a diagram showing an example of a surgical field image, an ultrasound image, and a superimposed image.
FIG. 8 is a diagram for describing a method of deriving a relative positional relationship.
FIG. 9 is a diagram for describing a method of estimating first position and orientation information.
FIG. 10 is a diagram showing an example of a surgical field image, an ultrasound image, and a superimposed image.
FIG. 11 is a diagram for describing processing of a determination unit.
FIG. 12 is a flowchart showing an example of medical support processing.
FIG. 13 is a diagram showing an example of a functional configuration of the medical support device according to a second embodiment.
FIG. 14 is a diagram for describing a method of estimating first position and orientation information using drive information.
FIG. 15 is a diagram for describing a method of performing transformation into an endoscope coordinate system.
FIG. 16 is a diagram for describing a method of estimating first position and orientation information using a movement amount.
FIG. 17 is a diagram for describing a method of estimating first position and orientation information using variation information.
FIG. 18 is a diagram showing an example of a functional configuration of the medical support device according to a third embodiment.
FIG. 19 is a diagram for describing a method of estimating first position and orientation information using an auxiliary image.
FIG. 20 is a diagram for describing a method of estimating first position and orientation information using an auxiliary image.
FIG. 21 is a diagram showing a relationship in a position and an orientation between a marker and an ultrasound probe.
FIG. 22 is a diagram showing an example of a surgical field image, an ultrasound image, and a superimposed image.
FIG. 23 is a diagram for describing tracking of an auxiliary endoscope.
FIG. 24 is a diagram for describing a method of estimating first position and orientation information using parallax information.
FIG. 25 is a diagram for describing a method of estimating first position and orientation information using an auxiliary image.
FIG. 26 is a diagram for describing a method of estimating first position and orientation information using an auxiliary image.

### DESCRIPTION OF EMBODIMENTS

Hereinafter, an example of an embodiment of the disclosed technology will be described with reference to the drawings. The same or equivalent components and parts in the respective drawings are denoted by the same reference numerals, and the duplicated description will be omitted. In addition, dimensional ratios in the drawings are exaggerated for convenience of description and may be different from the actual ratios.

### First Embodiment

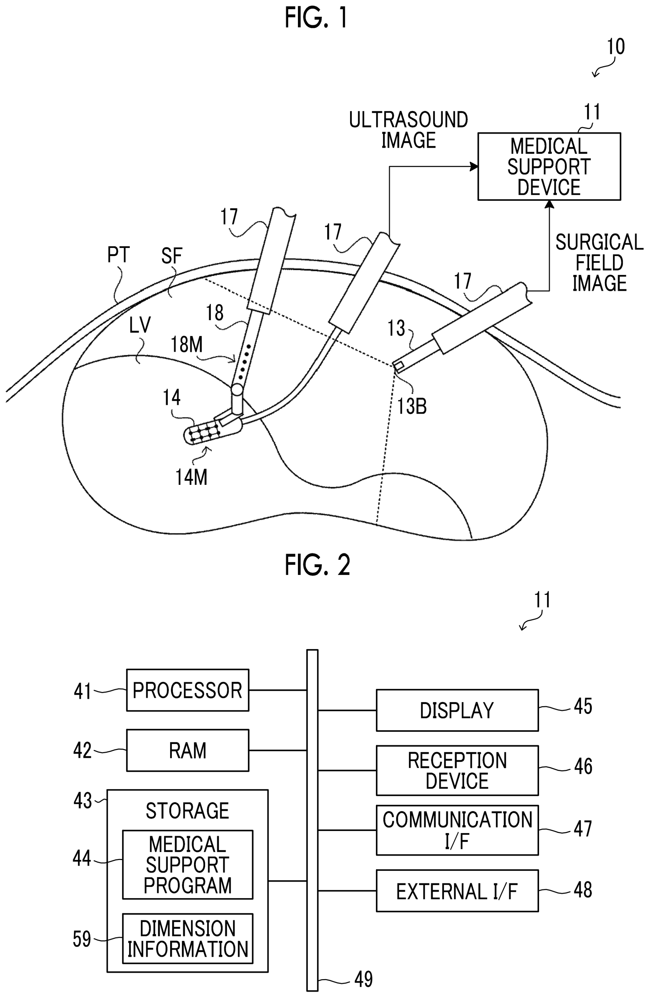

An example of a medical support system 10 to which a medical support device 11 according to the present embodiment is applied will be described with reference to FIG. 1. The medical support system comprises the medical support device 11, an endoscope 13, and an ultrasound probe 14. The medical support device 11 is communicably connected to the endoscope 13 and the ultrasound probe 14.

As an example, the medical support system is used in a case where a surgery using an endoscopic surgery support robot (hereinafter, referred to as a robotic surgery) is performed on a patient PT. The robotic surgery involves creating a plurality of small holes in a body of the patient PT, inserting various medical instruments connected to robotic arms through the holes, and having a surgeon remotely control the instruments via a dedicated console or the like.

The medical support system 10 provides a medical staff including a surgeon with a field of view of a surgical field SF inside the body of the patient PT, and with support information for supporting medical care such as a surgery and an examination. The support information, as described below, includes a superimposed image 26 obtained by superimposing and displaying an ultrasound image 22 on a surgical field image 21. Such a medical support system 10 has a function of providing the support information in real time during a surgery, and is therefore also called a surgical navigation system or the like.

FIG. 1 shows a state in which the endoscope 13, the ultrasound probe 14, and forceps 18 are inserted into the abdomen of the patient PT. The various medical instruments are inserted into the body via a trocar 17. In addition, pneumoperitoneum is performed by injecting carbon dioxide into the abdominal cavity. The trocar 17 is an insertion tool having an insertion hole into which various medical instruments are inserted and a valve provided in the insertion hole to prevent gas leakage.

In the example of FIG. 1, the forceps 18 grips the ultrasound probe 14. A position and an orientation of the ultrasound probe 14 are controlled by operating a robot arm (not shown) connected to the forceps 18. The medical instrument used in the medical support system 10 is not limited to the endoscope 13, the ultrasound probe 14, and the forceps 18. For example, a biopsy needle, a tissue sampling needle, an injection needle, a snare, an electric scalpel, or a high-frequency knife may be applied.

The endoscope 13 optically images a surgical field SF including a target part (in this example, a liver LV) inside the body of the patient PT by using a camera. The surgical field SF is a space that spreads in a body cavity defined by an organ and a body wall inside the body. Specifically, the endoscope 13 has an insertion part to be inserted into the body of the patient PT. The camera 13B and a light source (for example, a light emitting diode (LED)) for illumination are incorporated in a distal end part of the insertion part. The endoscope 13 is, for example, a rigid endoscope in which the insertion part is rigid, and is often used for abdominal cavity observation, so that the endoscope 13 is also called a laparoscope.

The camera 13B has an image sensor such as a charge coupled device (CCD) image sensor and a complementary metal oxide semiconductor (CMOS) image sensor, and an imaging optical system including a lens that forms a subject image on an imaging surface of the image sensor. The image sensor is, for example, an image sensor capable of capturing a color image. The endoscope 13 is connected to an image processing processor for an endoscope (not shown). This image processing processor performs signal processing on an imaging signal output by the image sensor to generate a surgical field image 21 of the surgical field SF inside the body. The surgical field image 21 captured by the endoscope 13 is transmitted to the medical support device 11 in real time via the image processing processor for an endoscope. The camera 13B is an example of a "camera" of the present disclosure.

As illumination light for the endoscope 13, for example, visible light such as white light is used. As the illumination light of the endoscope 13, special light such as ultraviolet light and infrared light may be used. As the special light, for example, light restricted to a specific wavelength such as short-wavelength narrow-band light obtained by narrowing down light in a short wavelength range such as an ultraviolet range may be used. The surgical field image 21 is a video of the surgical field SF illuminated with illumination light, and more specifically, is a video based on light reflected from the illumination light near the surface of the surgical field SF. Therefore, in the surgical field image 21, a structure present in the vicinity of a surface layer of the target part can be visualized, but it is difficult to observe an internal structure.

The ultrasound probe 14 transmits an ultrasound wave to the target part and detects an electric signal corresponding to an ultrasound echo reflected from the target part, thereby capturing an ultrasound image 22 of the target part. Specifically, the ultrasound probe 14 has an ultrasound transducer. The ultrasound transducer transmits an ultrasound wave to the target part and receives an ultrasound echo reflected from the target part. The ultrasound probe 14 is connected to an image processing processor for an ultrasound probe (not shown). This image processing processor performs image reconstruction processing based on an electric signal corresponding to the ultrasound echo received by the ultrasound transducer so that an ultrasound image corresponding to the electric signal is generated. Through the image reconstruction processing, the ultrasound image 22 showing an internal structure of the target part scanned by the ultrasound probe 14 is generated. The ultrasound image 22 captured by the ultrasound probe 14 is transmitted to the medical support device 11 in real time via the image processing processor for an ultrasound probe.

The ultrasound probe 14 is, for example, a convex type that radially transmits ultrasound waves, and acquires a fan-shaped ultrasound image 22 with the ultrasound transducer as a base point. A plurality of the ultrasound images 22 are captured along a scanning direction by performing the scanning with the ultrasound probe 14. The ultrasound image 22 is a so-called brightness (B)-mode image in which an internal structure from a surface layer to a deep layer where the ultrasound wave reaches the target part is visualized as brightness information. The ultrasound image 22 visualizes an internal structure of the target part that cannot be observed in the surgical field image 21 obtained by optical imaging.

The medical support device 11 acquires the surgical field image 21 from the endoscope 13, and acquires the ultrasound image 22 from the ultrasound probe 14. In addition, the medical support device 11 generates a superimposed image 26 by superimposing the ultrasound image 22 on the surgical field image 21, and controls a display to display the superimposed image 26. The field of view inside the body of the patient PT is provided to the medical staff through a screen of the display. In the robotic surgery, the surgeon performs a surgery, an examination, and the like by operating the robot arm via the dedicated console while referring to these images.

Hereinafter, a detailed configuration of the medical support device 11 will be described. FIG. 2 shows an example of a hardware configuration of the medical support device 11. The medical support device 11 comprises a processor 41, a random access memory (RAM) 42, a storage 43, a display 45, a reception device 46, a communication interface (I/F) 47, and an external I/F 48. These units are connected to a bus 49 such as a system bus and a control bus, and can communicate with each other.

The processor 41 is, for example, a central processing unit (CPU), and integrally controls the respective units of the medical support device 11 in accordance with a control program and executes various types of processing in accordance with various types of application programs. The processor 41 is an example of a "processor" of the present disclosure.

The RAM 42 is a memory that transitorily stores information, and is used as a work memory by the processor 41. Examples of the RAM 42 include a dynamic random access memory (DRAM) and a static random access memory (SRAM).

The storage 43 is a non-volatile storage device that stores various programs, various parameters, and the like. Examples of the storage 43 include a hard disk drive (HDD) and a solid state drive (SSD). The storage 43 stores a medical support program 44 for causing a computer to function as the medical support device 11.

In addition, the storage 43 stores dimension information 59. The dimension information 59 includes information about a geometric size and shape of a medical instrument (the ultrasound probe 14, the forceps 18, and the like) to which a marker described below is assigned. This is, for example, information indicating a length, a width, a diameter, a height, a thickness, and a structural feature (linear or curved shape) of the entire medical instrument and/or a specific part (such as distal end part) of the medical instrument. In addition, the dimension information 59 includes information about a shape of the marker and placement of the marker on the medical instrument. This is, for example, information indicating how the marker is provided in an axial direction and a circumferential direction of the medical instrument.

In addition to the surgical field image 21, the ultrasound image 22, and the superimposed image 26, various types of information are displayed on the display 45. Examples of the display 45 include a liquid crystal display and an electro-luminescence (EL) display. The number of the displays 45 is not particularly limited, and may be one or more.

The reception device 46 includes a keyboard, a mouse, and the like (not shown), and receives an instruction from the operator. That is, the medical support device 11 is operated by an operator such as the medical staff through the reception device 46. The reception device 46 may be a device that receives touch input, such as a touch panel, a device that receives voice input, such as a microphone, a device that receives gesture input, such as a camera, or the like.

The communication I/F 47 is connected to a network (not shown) such as a local area network (LAN) and/or a wide area network (WAN), and performs transmission control in accordance with a communication protocol defined in various types of wired or wireless communication standards. The external I/F 48 is, for example, a universal serial bus (USB) interface, and is used for connection to peripheral devices such as a printer and a memory card. As the medical support device 11, for example, a personal computer, a server computer, a smartphone, a tablet terminal, or a wearable terminal can be applied as appropriate.

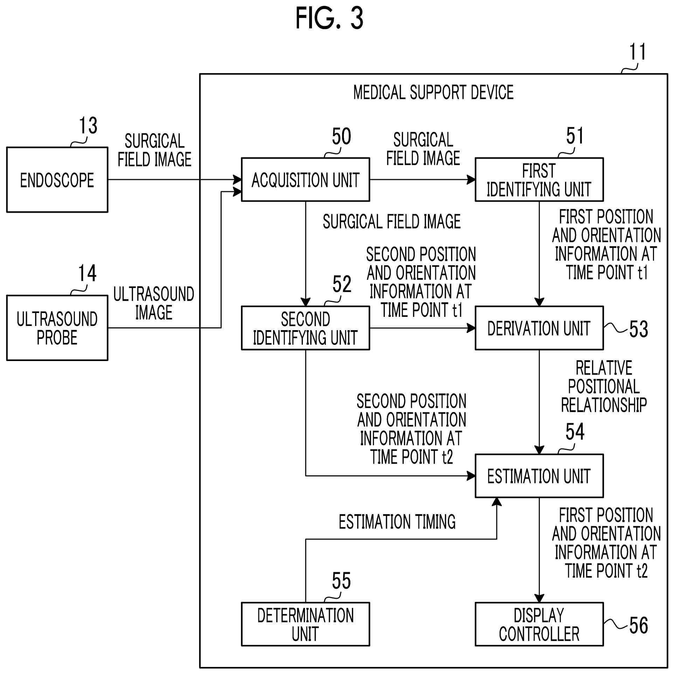

Next, a functional configuration of the medical support device 11 will be described. FIG. 3 is a block diagram showing an example of the functional configuration of the medical support device 11. The medical support device 11 comprises an acquisition unit 50, a first identifying unit 51, a second identifying unit 52, a derivation unit 53, an estimation unit 54, a determination unit 55, and a display controller 56 as functional units. The processor 41 reads out the medical support program 44 from the storage 43 and executes the medical support program 44 on the RAM 42, so that the processor 41 functions as each functional unit. The processor 41 operates as each functional unit to implement the medical support processing.

The acquisition unit 50 acquires a surgical field image 21 obtained by imaging, with the camera 13B, the surgical field SF including a first medical instrument to which the marker is assigned. For example, the acquisition unit 50 acquires the surgical field image 21 from a device including a processor of the endoscope 13 via the external I/F 48 or the communication I/F 47. The processor of the endoscope 13 may be provided in the medical support device 11.

In addition, the acquisition unit 50 acquires the ultrasound image 22 captured by the ultrasound probe 14. For example, the acquisition unit 50 acquires the ultrasound image 22 from the device including the processor of the ultrasound probe 14 via the external I/F 48 or the communication I/F 47. The processor of the ultrasound probe 14 may be provided in the medical support device 11.

The first identifying unit 51 identifies first position and orientation information indicating a position and an orientation of the first medical instrument based on the marker included in the surgical field image 21. Hereinafter, an example will be described in which the ultrasound probe 14 is used as an example of the "first medical instrument" and a marker 14M assigned to the ultrasound probe 14 is used as an example of the "marker".

The marker 14M is a marker that can be recognized from the surgical field image 21 optically captured by the camera 13B, and is assigned to the ultrasound probe 14. In addition, information about a shape of the marker 14M and placement of the marker 14M on the ultrasound probe 14 is known, and is stored in advance in the storage 43 as the dimension information 59.

Specifically, the first identifying unit 51 detects the marker 14M by searching for a morphological feature of the marker 14M from the surgical field image 21. For example, the first identifying unit 51 may detect the marker 14M by using an image processing method such as pattern matching. In addition, for example, the first identifying unit 51 may detect the marker 14M by using an artificial intelligence (AI) technology using a machine learning model. As such a machine learning model, for example, a neural network model such as a CNN that has been trained in advance by using the surgical field image 21 as an input and a region of the marker 14M in the input surgical field image 21 as an output can be applied.

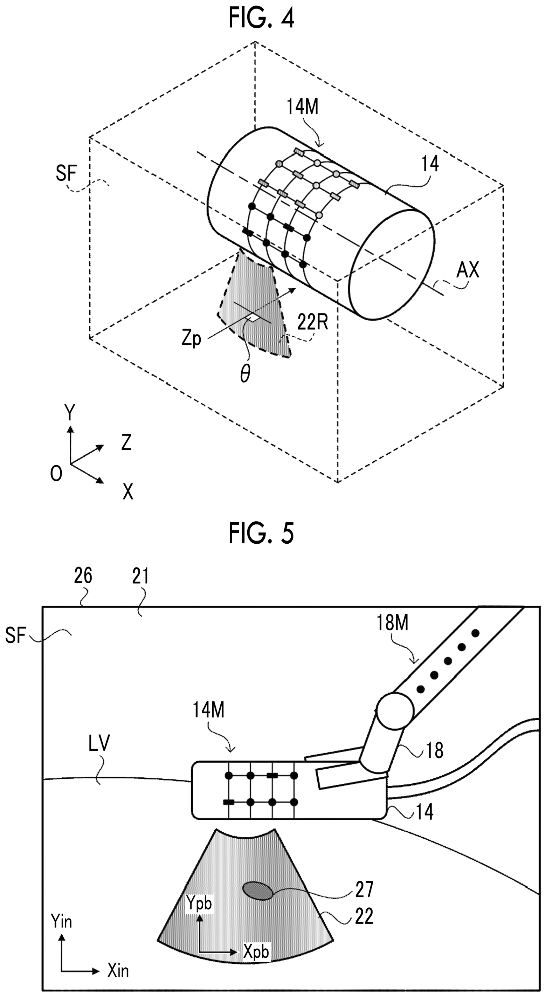

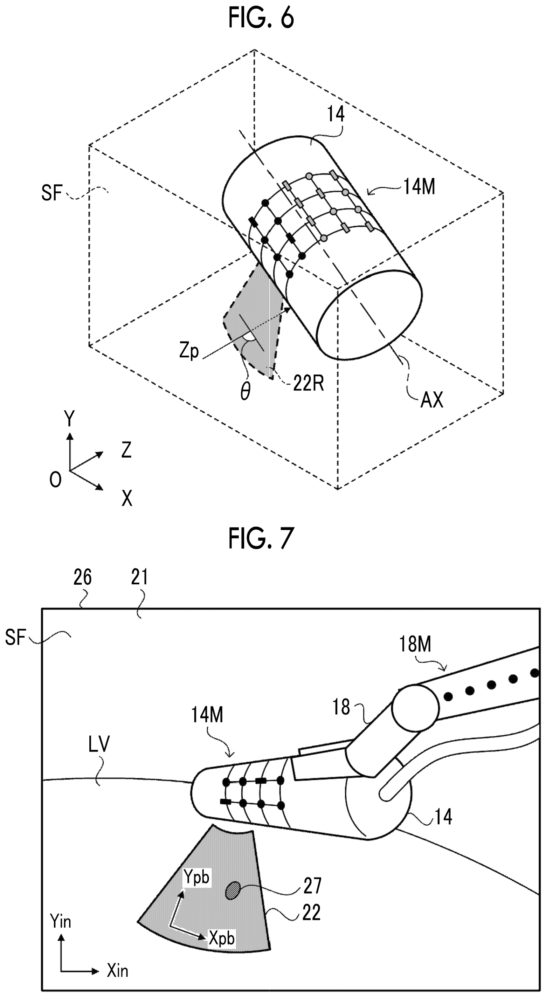

Then, the first identifying unit 51 identifies the first position and orientation information indicating the position and the orientation of the ultrasound probe 14 based on the detected marker 14M. A method of identifying the first position and orientation information will be described with reference to FIGS. 4 to 7. FIGS. 4 and 6 are diagrams conceptually showing a distal end part of the ultrasound probe 14 in the surgical field SF defined as a three-dimensional space, each of which assumes a different position and orientation. FIG. 5 shows a surgical field image 21 captured in a case where the position and the orientation of the distal end part of the ultrasound probe 14 are in the state of FIG. 4. FIG. 7 shows a surgical field image 21 captured in a case where the position and the orientation of the distal end part of the ultrasound probe 14 are in the state of FIG. 6.

As an example, the marker 14M is a marker of a lattice pattern composed of a first line extending in a direction of an axis AX of the distal end part of the ultrasound probe 14 and a second line orthogonal to the direction of the axis AX of the distal end part and formed in the circumferential direction along an outer peripheral surface of the distal end part. A circular symbol or a rectangular symbol is assigned to each intersection in the lattice pattern. In each drawing, the marker 14M may be simplified or omitted.

The marker 14M may include at least one of a line forming the lattice pattern or a symbol disposed at each intersection of the lattice pattern. In addition, the symbol on the marker 14M can have any shape, and may be represented by figures such as a triangle, a polygon, a star shape, and various marks, or may be represented by a character or the like. In addition, as the marker 14M, a two-dimensional code and a geometric pattern (for example, ArUco and ChArUco) may be used.

In the surgical field SF of the three-dimensional space shown in FIGS. 4 and 6, a Z-axis is a direction parallel to an imaging optical axis of the camera 13B. In addition, an X-Y plane in the surgical field SF is a plane parallel to an imaging plane (Xin-Yin plane) of the camera 13B and is orthogonal to the imaging optical axis. That is, the surgical field image 21 corresponds to a projection image obtained by projecting the surgical field SF from one viewpoint O. In FIGS. 4 and 6, among the symbols constituting the marker 14M, a symbol reflected in the surgical field image 21 is shown in a dark color, and a symbol not reflected in the surgical field image 21 is shown in a light color.

The orientation of the ultrasound probe 14 is detected, for example, as a direction of the axis AX of the distal end part of the ultrasound probe 14 in the surgical field SF. The direction of the axis AX is represented by, for example, an inclined angle with respect to each of an X-axis, a Y-axis, and a Z-axis.

For example, FIG. 4 shows a state in which the axis AX of the distal end part of the ultrasound probe 14 is parallel to the X-axis in the surgical field SF of the three-dimensional space. In this case, as shown in FIG. 5, the marker 14M shown in the surgical field image 21 has orthogonal lines of the lattice pattern parallel to the X-axis and Y-axis. In addition, the symbols forming the lattice pattern are shown in the surgical field image 21 at equal intervals.

On the other hand, FIG. 6 shows a state in which the axis AX of the distal end part of the ultrasound probe 14 is inclined with respect to the depth direction parallel to the imaging optical axis in the surgical field SF of the three-dimensional space. In this case, as shown in FIG. 7, in the marker 14M shown in the surgical field image 21, the farther the marker 14M is from the camera 13B in the depth direction, the shorter the line extending in the circumferential direction of the lattice pattern is, and the shorter the interval between the symbols forming the lattice pattern is.

As described above, the form of the marker 14M shown in the surgical field image 21 changes depending on the orientation of the ultrasound probe 14. The first identifying unit 51 identifies the direction of the axis AX of the distal end part of the ultrasound probe 14 as the orientation based on the form (projected lattice pattern or the like) of the marker 14M that changes in this way in the surgical field image 21.

In addition, in a case where the position of the ultrasound probe 14 is changed in the surgical field SF, the position of the marker 14M shown in the surgical field image 21 is also changed. The first identifying unit 51 may identify the position of the ultrasound probe 14 in the surgical field SF based on the position of the marker 14M. The position of the ultrasound probe 14 is detected, for example, as position coordinates (X,Y,Z) of a predetermined reference point (for example, a distal end position) in the ultrasound probe 14.

An imaging distance from the camera 13B to the marker 14M (that is, a coordinate in a Z-axis direction) can be calculated based on a focal length of the camera 13B and the size of the marker 14M shown in the surgical field image 21. Specifically, by associating the size of the marker 14M detected from the surgical field image 21 with the information on the marker 14M included in the dimension information 59, the amount of change in the projection scale can be estimated, and the imaging distance can be calculated. For example, the apparent size of the marker 14M is smaller as the distal end part is farther from the camera 13B and is larger as the distal end part is closer to the camera 13B. The first identifying unit 51 identifies the imaging distance by using such a geometric relationship.

In addition, FIGS. 4 and 6 also show an angle θ representing a direction of the ultrasound image 22. The angle θ is at its minimum in a case where the imaging optical axis (Z-axis) of the camera 13B is parallel to a display surface (Xpb-Ypb plane) of the ultrasound image 22, and is at its maximum in a case where the imaging optical axis is a normal line. In FIGS. 4 and 6, for ease of understanding, a straight line Zp parallel to the Z-axis passing through the display surface of the ultrasound image 22 is shown. The angle θ can be derived based on the first position and orientation information and the dimension information 59.

The second identifying unit 52 acquires second position and orientation information indicating a position and an orientation of a second medical instrument connected to the first medical instrument. The term "second medical instrument connected to the first medical instrument" means a state in which the first medical instrument and the second medical instrument are combined by a connecting means such as gripping, engagement, and attachment and are operated substantially integrally. Hereinafter, an example will be described in which the forceps 18 are used as an example of the "second medical instrument".

The second position and orientation information acquired by the second identifying unit 52 includes second position and orientation information at the same time point as a time point at which the first position and orientation information is identified and second position and orientation information at at least one time point thereafter. Hereinafter, a time point at which both the first position and orientation information and the second position and orientation information are acquired is referred to as a "first time point t1", and a time point after the first time point t1 is referred to as a "second time point t2". That is, the acquisition unit 50 acquires the surgical field image 21 at the first time point t1. The first identifying unit 51 identifies the first position and orientation information at the first time point t1. The second identifying unit 52 acquires the second position and orientation information at the first time point t1 and the second time point t2. The "same time point" does not mean the exact same time, but rather a concept that includes a range of times that can be considered substantially the same, taking into account measurement errors, communication delays, and the like.

A method of identifying the second position and orientation information is not particularly limited. In addition, a method of identifying the second position and orientation information at the first time point t1 and a method of identifying the second position and orientation information at the second time point t2 may be the same or different from each other.

For example, it is assumed that the surgical field image 21 includes the forceps 18 to which a marker 18M is assigned. In this case, the second identifying unit 52 may identify the second position and orientation information at at least one of the first time point t1 or the second time point t2 based on the marker 18M included in the surgical field image 21. The method of identifying the second position and orientation information from the marker 18M is the same as a method of identifying the first position and orientation information from the marker 14M.

In addition, for example, it is assumed that the surgical field image 21 includes the forceps 18. In this case, the second identifying unit 52 may identify the second position and orientation information at at least one of the first time point t1 or the second time point t2 based on a characteristic shape of the forceps 18 included in the surgical field image 21. The term "characteristic shape" means a shape that allows the forceps 18 to be distinguished in appearance from other medical instruments and various structures such as organs. For example, characteristic contours, dimensions, colors, and reflection properties can be applied as appropriate. This characteristic shape is included in the dimension information 59 in advance.

In addition, for example, the second identifying unit 52 may identify the second position and orientation information at at least one of the first time point t1 or the second time point t2 based on drive information of the forceps 18. The drive information will be described below.

The derivation unit 53 derives a relative positional relationship between the first medical instrument and the second medical instrument based on the first position and orientation information and the second position and orientation information at the first time point t1. The "relative positional relationship" is a general term for parameters indicating a distance or vector in spatial coordinates between medical instruments, as well as a difference in orientation. In a case where the forceps 18 and the ultrasound probe 14 are connected to each other, the relative positional relationship is basic information required to handle both the forceps 18 and the ultrasound probe 14 integrally.

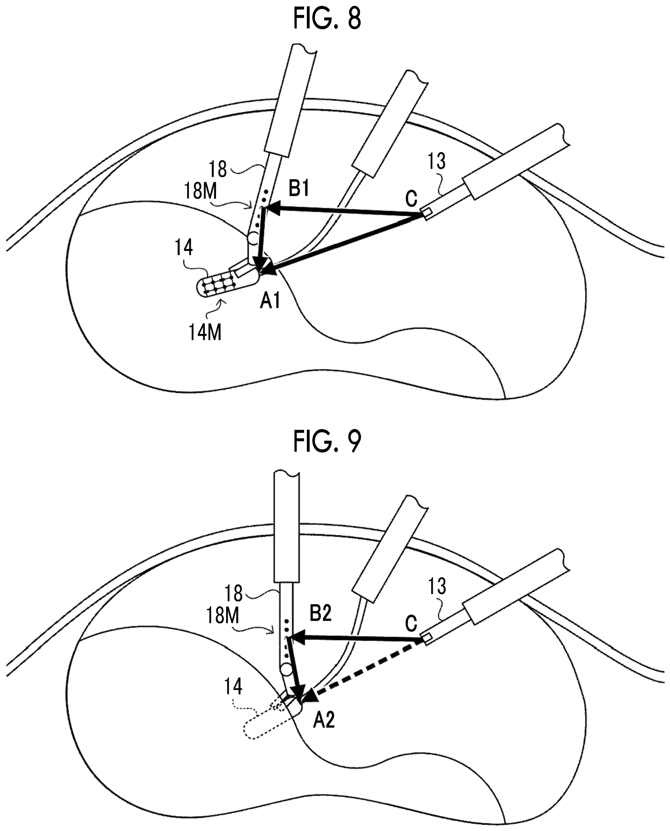

The relative positional relationship will be described with reference to FIG. 8. A position of the ultrasound probe 14 in the surgical field SF at the first time point t1 is denoted by A1, a position of the forceps 18 is denoted by B1, and a position of the endoscope 13 (that is, a viewpoint of the surgical field image 21) is denoted by C.

A vector CA1 from the position C to the position A1 can be calculated based on the first position and orientation information at the first time point t1. A vector CB1 from the position C to the position B1 can be calculated based on the second position and orientation information at the first time point t1. A vector B1A1 from the position B1 to the position A1 can be obtained by using the following equation according to a basic rule of vector calculation.$Vector B 1 A 1 = vector CA 1 - vector CB 1$

Here, it is assumed that a relative positional relationship between the forceps 18 and the ultrasound probe 14 is fixed at each time point. For example, even in a case where the forceps 18 perform an operation such as movement and rotation as a whole, a connection portion with the ultrasound probe 14 is fixed and does not move. In this case, the vector B1Al calculated at the first time point t1 remains the same as a relative vector from the position of the forceps 18 to the position of the ultrasound probe 14 even at a subsequent time point. In other words, the relative vector connecting the ultrasound probe 14 and the forceps 18 can be treated as an invariant.

The estimation unit 54 estimates the first position and orientation information at the second time point t2 based on the relative positional relationship and the second position and orientation information at the second time point t2. A method of estimating the first position and orientation information at the second time point t2 will be described with reference to FIG. 9. A position of the ultrasound probe 14 in the surgical field SF at the second time point t2 is denoted by A2, a position of the forceps 18 is denoted by B2, and a position of the endoscope 13 is denoted by C.

A vector CB2 from the position C to the position B2 can be calculated based on the second position and orientation information at the second time point t2. Since the relative vector B1A1 does not change, the relative vector B1A1 calculated at the first time point t1 can be regarded as a vector B2A2 from the position B2 to the position A2 at the second time point t2. A vector CA2 from the position C to the position A2 can be obtained by using the following equation according to a basic rule of vector calculation. $\begin{matrix}Vector CA 2 = vector CB 2 + vector B 2 A 2 \\ = vector CB 2 + relative vector B 1 A 1\end{matrix}$

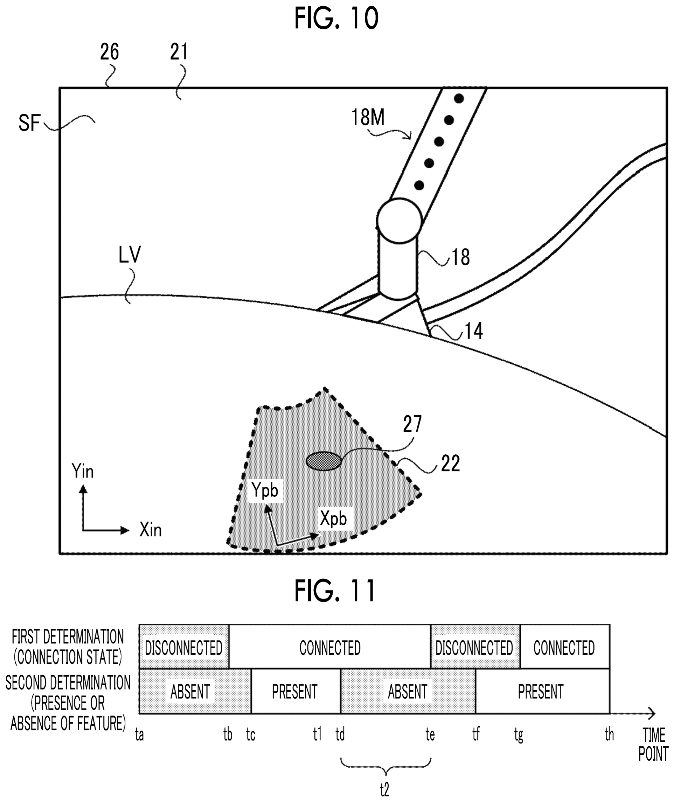

As described above, even in a case where the position B2 of the forceps 18 is known, the position A2 of the ultrasound probe 14 can be estimated by using the relative vector B1A1. Therefore, as shown in FIG. 10, even in a case where the ultrasound probe 14 disappears or is blocked from the surgical field image 21 by operating the forceps 18 or the like and becomes unreadable, the first position and orientation information can be appropriately estimated.

During an actual surgery, the relative positional relationship does not always remain unchanged, and the connection between the forceps 18 and the ultrasound probe 14 may be released, causing the relative positional relationship to change. Therefore, the determination unit 55 may allow the estimation of the first position and orientation information based on the relative positional relationship only at a timing at which the relative positional relationship has not changed.

Specifically, the determination unit 55 determines whether or not an amount of variation between the relative positional relationship at the first time point t1 and the relative positional relationship at the second time point t2 is within a predetermined threshold value. The expression "amount of variation is within the threshold value" means a range in which a change in the relative positional relationship can be substantially ignored even in consideration of a measurement error, a device tolerance, and the like. That is, the determination unit 55 determines whether or not the connection portion between the forceps 18 and the ultrasound probe 14 is fixed and the relative positional relationship has not changed.

The amount of variation in the relative positional relationship may be detected from the surgical field image 21, for example. In addition, for example, the amount of variation may be detected based on the drive information of the forceps 18. The drive information will be described below.

In a case where the amount of variation is within the threshold value, the estimation unit 54 estimates the first position and orientation information at the second time point t2 based on the relative positional relationship and the second position and orientation information at the second time point t2. That is, the estimation unit 54 estimates the first position and orientation information from the relative positional relationship at a timing at which it is determined that the relative positional relationship between the forceps 18 and the ultrasound probe 14 has not substantially changed.

The display controller 56 performs control of superimposing and displaying the ultrasound image 22 on the surgical field image 21. Specifically, the display controller 56 estimates a position and an orientation of the ultrasound transducer in the surgical field SF based on the first position and orientation information and the dimension information 59. A linear distance between a reference point of the position and the orientation indicated by the first position and orientation information and the ultrasound transducer and an inclined angle of the ultrasound transducer with respect to the direction of the axis AX are known information defined as the dimension information 59. By using the dimension information 59, the position and the orientation of the ultrasound transducer in the surgical field SF can be estimated from the first position and orientation information.

The display controller 56 estimates the imaging range of the ultrasound image 22 in the surgical field SF based on the estimated position and orientation of the ultrasound transducer. The imaging range of the ultrasound image 22 corresponds to the position and the orientation of the ultrasound transducer. For example, the imaging range of the ultrasound image 22 captured by the convex type ultrasound transducer is a fan-shaped range that spreads radially from the ultrasound transducer as a base point. In FIGS. 4 and 6, an imaging range 22R of the ultrasound image 22 in the surgical field SF is shown.

Then, the display controller 56 generates the superimposed image 26 by superimposing the ultrasound image 22 on a region (Xin-Yin plane) in the surgical field image 21 corresponding to the imaging range (Xpb-Ypb plane) of the ultrasound image 22 in the surgical field SF. In the examples of FIGS. 4 and 5, the imaging range (Xpb-Ypb plane) of the ultrasound image 22 in the surgical field SF and the screen (Xin-Yin plane) of the surgical field image 21 in the surgical field SF are parallel to each other. On the other hand, in the examples of FIGS. 6 and 7, the imaging range (Xpb-Ypb plane) of the ultrasound image 22 in the surgical field SF and the screen (Xin-Yin plane) of the surgical field image 21 in the surgical field SF are not parallel to each other. The display controller 56 performs deformation such as projective transformation, affine transformation, movement, rotation, enlargement, and reduction on the ultrasound image 22 in consideration of a difference in coordinate systems between the surgical field image 21 and the ultrasound image 22, and then generates the superimposed image 26 superimposed on the surgical field image 21.

In addition, in the example of FIG. 10, the ultrasound probe 14 is blocked by the liver LV and is not shown in the surgical field image 21, but, with the medical support device 11 according to the present embodiment, the first position and orientation information at the second time point t2 can be estimated. Therefore, the display controller 56 generates the superimposed image 26 by superimposing the ultrasound image 22 on the surgical field image 21 based on the estimated position and orientation of the ultrasound transducer in the same manner as in the examples of FIGS. 4 to 7.

With the superimposed image 26, for example, even in a case where the position and the orientation of the ultrasound probe 14 cannot be visually recognized or are difficult to visually recognize in the surgical field image 21 due to blocking by an organ or the like and a positional relationship with the camera 13B, the position and the orientation can be easily grasped. Therefore, it is easy to adjust the position and the orientation of the ultrasound probe 14 so that a desired region (for example, the tumor 27) is reflected in the ultrasound image 22, and it is possible to contribute to the improvement of the operability.

### Modification Example 1

In addition to the above-described embodiment, the determination unit 55 may determine a timing at which the relative positional relationship is derived, that is, a timing at which the first time point t1 is set. As the timing at which the relative positional relationship is derived, a timing at which the connection between the forceps 18 and the ultrasound probe 14 is completed and the positions and the orientations of the forceps 18 and the ultrasound probe 14 can be identified from the surgical field image 21 is preferable.

Specifically, the determination unit 55 may perform a first determination of whether or not the forceps 18 are connected to the ultrasound probe 14. For example, the determination unit 55 may perform the first determination based on the surgical field image 21. In addition, for example, the determination unit 55 may perform the first determination based on the drive information. The drive information will be described below.

In addition, the determination unit 55 may perform a second determination of whether or not the marker 14M and a feature portion of the forceps 18 are included in the surgical field image 21. The term "feature portion" means a portion that allows the forceps 18 to be distinguished in appearance from other medical instruments and various structures such as organs, and is, for example, at least one of the marker 18M of the forceps 18 or the characteristic shape described above.

Then, the determination unit 55 may set a time point at which both the first determination and the second determination are affirmative, as the first time point t1. For example, the determination unit 55 may perform the first determination and the second determination over time, and, in a case where the second determination is negative, the determination unit 55 may set a time point at which both the immediately preceding first determination and second determination are affirmative, as the first time point t1.

FIG. 11 shows an example of a timing at which the first time point t1 is set. A period from a time point ta to a time point tb is not suitable for deriving the relative positional relationship because the connection between the forceps 18 and the ultrasound probe 14 is released and the relative positional relationship is indefinite. In a period from the time point tb to a time point tc, although the forceps 18 and the ultrasound probe 14 are connected, the position and the orientation of at least one of the forceps 18 or the ultrasound probe 14 cannot be identified from the surgical field image 21, and therefore, it is not suitable for deriving the relative positional relationship. As will be described below, the second position and orientation information of the forceps 18 can also be identified based on the drive information, so that it is not impossible to derive the relative positional relationship in a period from the time point tb to the time point tc.

A period from the time point tc to a time point td is suitable for deriving the relative positional relationship because the first position and orientation information and the second position and orientation information can be identified from the surgical field image 21. At the time point td, the second determination is switched to a negative determination, so that the time point immediately before the time point td is set as the first time point t1. Any time point within the period from the time point tc to the time point td can be set as the first time point t1, but, in consideration of a slight variation of the relative positional relationship, it is optimal to select the time point immediately before the time point td.

In a period from the time point td to a time point te, although the second determination is negative, the first determination is affirmative, and therefore, the first position and orientation information can be estimated based on the relative positional relationship derived at the time point t1. That is, in the period from the time point td to the time point te, the time point t2 is set. On the other hand, at the time point te, the first determination is switched to a negative determination, so that it is assumed that the connection between the forceps 18 and the ultrasound probe 14 is released and the relative positional relationship has changed. Therefore, a period from the time point te to a time point tf is not suitable for estimating the first position and orientation information based on the relative positional relationship, and is also not suitable for deriving the relative positional relationship.

In a period from the time point tf to a time point tg, the first determination is still negative, and it is assumed that the relative positional relationship between the forceps 18 and the ultrasound probe 14 is not invariant. Therefore, even in a case where the second determination is affirmative, the period is not suitable for estimating the first position and orientation information based on the relative positional relationship, and is also not suitable for deriving the relative positional relationship. In a period from the time point tg to a time point th, the first determination and the second determination are affirmative, so that the first position and orientation information and the second position and orientation information can be identified from the surgical field image 21, and the period is suitable for deriving the relative positional relationship. Since the first position and orientation information can be directly identified from the surgical field image 21 in the period from the time point tc to the time point td and the period from the time point tg to the time point th, the estimation based on the relative positional relationship is not necessary.

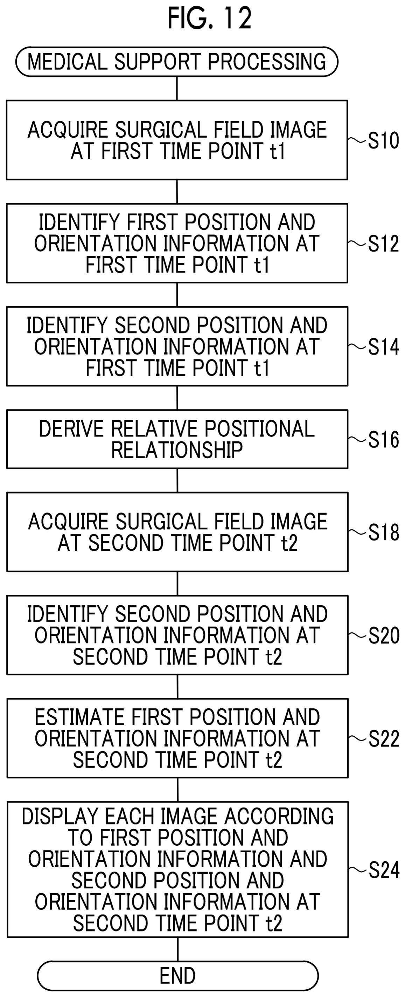

Next, an operation of the medical support device 11 according to the present embodiment will be described with reference to FIG. 12. In the medical support device 11, the medical support processing shown in FIG. 12 is executed by the processor 41 executing the medical support program 44. This processing is executed, for example, in a case where a user gives an instruction to start execution via the reception device 46.

In step S10, the acquisition unit 50 acquires the surgical field image 21 at the first time point t1. In step S12, the first identifying unit 51 identifies, based on the marker 14M included in the surgical field image 21 at the first time point t1, first position and orientation information indicating a position and an orientation of the first medical instrument (for example, the ultrasound probe 14), the first position and orientation information being information at the first time point t1. In step S14, the second identifying unit 52 identifies second position and orientation information indicating a position and an orientation of the second medical instrument (for example, the forceps 18), the second position and orientation information being information at the first time point t1. In step S16, the derivation unit 53 derives a relative positional relationship between the first medical instrument and the second medical instrument based on the first position and orientation information and the second position and orientation information at the first time point t1.

In step S18, the acquisition unit 50 acquires the surgical field image 21 at the second time point t2. In step S20, the second identifying unit 52 identifies the second position and orientation information at the second time point t2. In step S22, the estimation unit 54 estimates the first position and orientation information at the second time point t2 based on the relative positional relationship and the second position and orientation information at the second time point t2. In step S24, the display controller 56 performs control of displaying each image on the display 45 according to the first position and orientation information and the second position and orientation information at the second time point t2.

As described above, the medical support device 11 according to the present embodiment comprises a processor, in which the processor is configured to acquire, at a first time point t1, a surgical field image 21 obtained by imaging, with a camera 13B, a surgical field SF including a first medical instrument (ultrasound probe 14) to which a marker 14M is assigned. The processor identifies, based on the marker 14M included in the surgical field image 21, first position and orientation information indicating a position and an orientation of the first medical instrument, the first position and orientation information being information at the first time point t1. The processor acquires second position and orientation information indicating a position and an orientation of a second medical instrument (forceps 18) connected to the first medical instrument, the second position and orientation information being information at the first time point t1 and a second time point t2. The processor derives a relative positional relationship between the first medical instrument and the second medical instrument based on the first position and orientation information and the second position and orientation information at the first time point t1. The processor estimates the first position and orientation information at the second time point t2 based on the relative positional relationship and the second position and orientation information at the second time point t2.

That is, with the medical support device 11 according to the present embodiment, even in a case where the first medical instrument is not included in the field of view of the surgical field image 21 due to the movement of the medical instrument during a surgery, the position and the orientation of the first medical instrument can be estimated. Therefore, general-purpose properties in a case of estimating the position and the orientation of the medical instrument can be improved.

### Second Embodiment

The medical support device 11 according to the present embodiment performs various types of processing based on drive information, in addition to the functions of the medical support device 11 according to the first embodiment. In the following description, a part of the description overlapping with the first embodiment will be omitted.

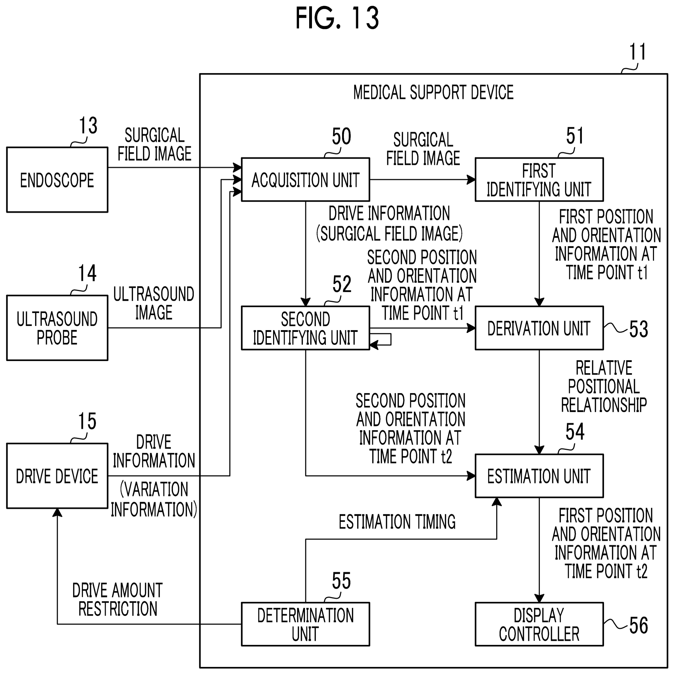

FIG. 13 is a block diagram showing an example of a functional configuration of the medical support device 11 according to the present embodiment. The medical support device 11 comprises an acquisition unit 50, a first identifying unit 51, a second identifying unit 52, a derivation unit 53, an estimation unit 54, a determination unit 55, and a display controller 56 as functional units. The processor 41 reads out the medical support program 44 from the storage 43 and executes the medical support program 44 on the RAM 42, so that the processor 41 functions as each functional unit.

The acquisition unit 50 acquires drive information of the second medical instrument (forceps 18). The term "drive information" is a general term for information used in a case of controlling various medical instruments with a drive device 15, and includes, for example, control information such as a movement vector, a target position coordinate, a rotation angle, an arm joint drive amount, and a gripping force. The term "target position coordinate" refers to information indicating, for example, a desired position to which the forceps 18 are to be moved. The term "movement vector" refers to vector information until the target position coordinate is reached. The drive information may include sensor information (for example, a joint angle and a load torque value) from a robot arm side in addition to commands input by the surgeon via the dedicated console.

For example, the acquisition unit 50 acquires the drive information from the drive device 15 via the external I/F 48 or the communication I/F 47. In a robotic surgery system, the drive device 15 is a device that integrates commands input by the surgeon via the dedicated console, feedback information from each actuator, and the like and that drives the position and the orientation of each medical instrument using the robot arm.

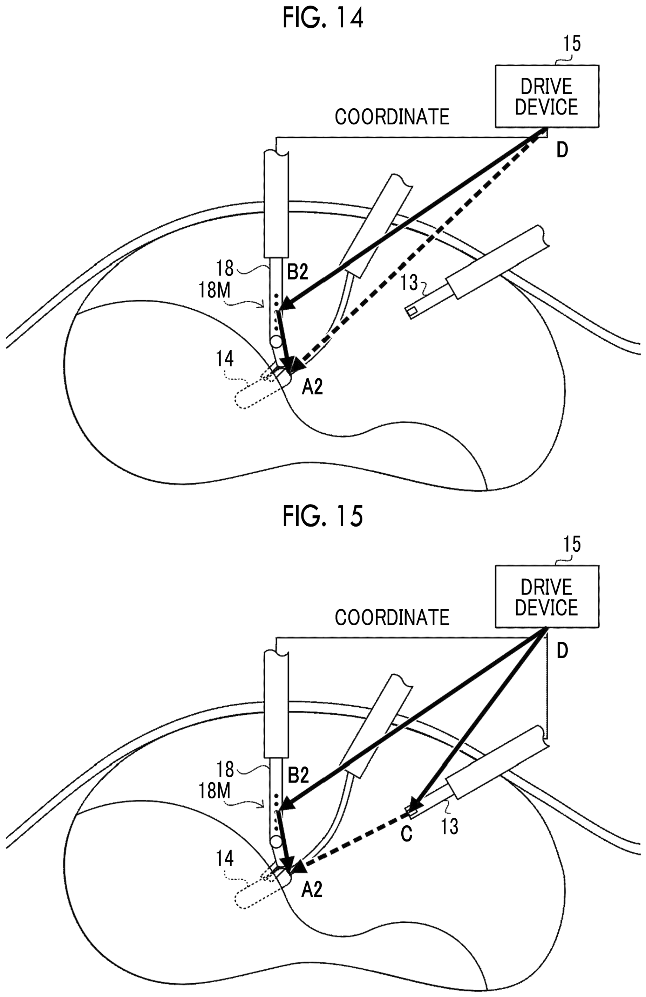

A method of estimating the first position and orientation information using the drive information and a method of identifying the second position and orientation information will be described with reference to FIG. 14. First, the first identifying unit 51 identifies the first position and orientation information at the first time point t1 based on the surgical field image 21. The second identifying unit 52 acquires the second position and orientation information at the first time point t1. The second position and orientation information at the first time point t1 may be identified based on the surgical field image 21 or may be identified based on the drive information. The derivation unit 53 derives a relative positional relationship between the first medical instrument and the second medical instrument based on the first position and orientation information and the second position and orientation information at the first time point t1.

For example, as shown in FIG. 8, a vector CA1 from the position C of the endoscope 13 to the position A1 of the ultrasound probe 14 in the surgical field SF at the first time point t1 is calculated. In addition, a vector CB1 from the position C to the position B1 of the forceps 18 is calculated. In addition, a relative vector B1A1 connecting the ultrasound probe 14 and the forceps 18 is calculated.

Next, the second identifying unit 52 identifies the second position and orientation information at the second time point t2 based on the second position and orientation information at the first time point t1 and the drive information. A position of the ultrasound probe 14 in the surgical field SF at the second time point t2 is denoted by A2, a position of the forceps 18 is denoted by B2, and a position of the drive device 15 is denoted by D. The "position D of the drive device 15" is a concept indicating an origin or a reference point in a coordinate system of the drive information. For example, in a case of controlling the robot arm, an installation position of a robot body or a reference position of each actuator may correspond to the position D. In a case where the coordinate system referred to by the medical support device 11 is different from the coordinate system handled internally by the drive device 15, it is necessary to hold parameters (scale, rotation matrix, and the like) for performing transformation between the two coordinate systems.

For example, it is assumed that the drive information is the target position coordinate of the forceps 18. In this case, the second identifying unit 52 identifies a vector DB2 from the position D of the drive device 15 to the position B2 of the forceps 18 at the second time point t2 based on the target position coordinate. Specifically, the second identifying unit 52 calculates a distance and a direction in which the forceps 18 is moved from the position D, as the vector DB2.

The estimation unit 54 estimates the first position and orientation information at the second time point t2 based on the relative positional relationship and the second position and orientation information at the second time point t2. A vector DA2 from the position D to the position A2 can be obtained by using the following equation according to a basic rule of vector calculation. $Vector DA 2 = vector DB 2 + relative vector B 1 A 1$

The first position and orientation information (vector DA2) in this case indicates a position and an orientation with respect to the position D of the drive device 15. That is, it indicates a value in a coordinate system different from the vector CA1 at the first time point t1. On the other hand, by considering the position C of the endoscope 13 with respect to the drive device 15, it is also transformed into the first position and orientation information in the coordinate system of the endoscope 13 in the same manner as at the first time point t1.

A method of transforming the vector DA2 into the coordinate system of the endoscope 13 will be described with reference to FIG. 15. First, the first identifying unit 51 identifies a vector DC from the position D of the drive device 15 to the position C of the endoscope 13 based on the drive information. The vector DC may be directly included in the drive information or may be indirectly calculated from position information and a joint angle of the robot arm connecting the position D and the position C. A vector CA2 from the position C to the position A2 can be obtained by using the following equation according to a basic rule of vector calculation. $Vector CA 2 = vector DA 2 - vector DC$

The drive information of the endoscope 13 may indicate a coordinate that does not necessarily match the position C that is the viewpoint of the surgical field image 21, such as a base end part of the endoscope 13. In this case, by calculating the position C based on the dimension information 59 of the endoscope 13 body or the like, the coordinate transformation aligned with the viewpoint of the camera 13B becomes possible. In addition, for example, the position C of the camera 13B may be derived by detecting an insertion length and a bending angle of the endoscope 13.

The determination unit 55 may restrict the drive device 15 such that the relative positional relationship between the ultrasound probe 14 and the forceps 18 does not change. Specifically, the determination unit 55 may restrict a drive amount of the forceps 18 such that an amount of variation in the relative positional relationship over time is within a predetermined threshold value, after the first time point t1. For example, while the ultrasound probe 14 is being gripped by the forceps 18, the determination unit 55 may invalidate control inputs whose variation amounts of the gripping position and the gripping force exceed a predetermined range. For example, an upper limit value for the joint angle and the movement amount of the forceps 18 may be set.

In addition, the determination unit 55 may determine whether or not the forceps 18 are connected to the ultrasound probe 14, and may implement the restriction of the drive amount of the forceps 18 in a case where the determination is affirmative. For example, the determination unit 55 may determine a contact state between the ultrasound probe 14 and the forceps 18 based on the surgical field image 21. In addition, for example, the determination unit 55 may determine the connection state in a case where it is detected that the forceps 18 is closed or it is detected that the forceps 18 are weighted with a gripping force of a certain or higher level, based on the drive information.

In addition, the determination unit 55 may receive designation as to whether or not to allow variation of the relative positional relationship after the first time point t1, and implement the restriction of the drive amount of the forceps 18 in a case where it is designated that the variation of the relative positional relationship is not allowed. For example, in a case where the surgeon inputs a command of "relative position fixation" from an operation panel, a button, or the like of the dedicated console for a robotic surgery, a gripping part of the forceps 18 may be locked, and the relative positional relationship between the forceps 18 and the ultrasound probe 14 may be fixed.

In a case where the first medical instrument is also driven by the drive device 15, it is preferable that the determination unit 55 also restrict the drive amount of the first medical instrument. This is because it is necessary to restrict the drive amounts of both the first medical instrument and the second medical instrument in order to fix the relative positional relationship between the first medical instrument and the second medical instrument. For example, it is assumed that the robot arm is also connected to the ultrasound probe 14 side, and that, in order to maintain the connection portion with the forceps 18, it is necessary to control the position and the orientation on the ultrasound probe 14 side. In this case, it is preferable that the determination unit 55 perform drive control of each medical instrument such that the relative positional relationship of each medical instrument is maintained within a desired range.

### Modification Example 2-1

In the above-described embodiment, a form has been described in which the coordinate information is used as the drive information, but the present disclosure is not limited to this. For example, the drive information may be information indicating the movement amount of the forceps 18. The term "movement amount" refers to vector information indicating a distance and a direction in which the forceps 18 have moved between respective time points.

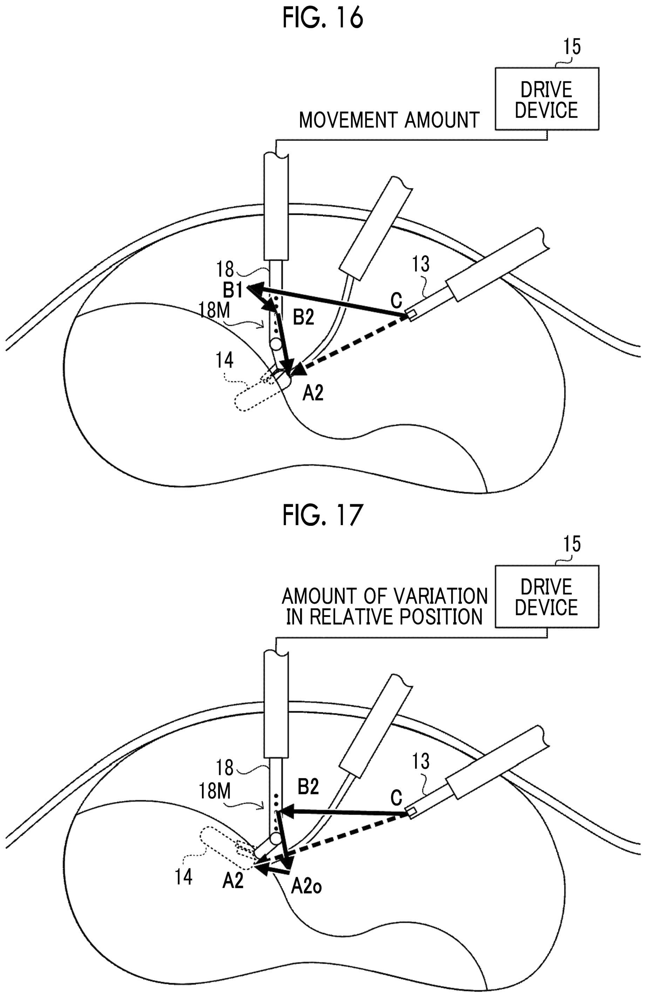

A method of estimating the first position and orientation information and a method of identifying the second position and orientation information in this case will be described with reference to FIG. 16. The acquisition unit 50 acquires drive information including the movement amount of the forceps 18 from the first time point t1 to the second time point t2.

The second identifying unit 52 acquires second position and orientation information at the first time point t1, the second position and orientation information being relative to the viewpoint (that is, the coordinate system of the endoscope 13) of the surgical field image 21. Specifically, the second identifying unit 52 acquires a vector CB1 from the position C of the endoscope 13 to the position B1 of the forceps 18 at the first time point t1. The movement amount of the forceps 18 is represented by a movement vector B1B2 from the position B1 of the forceps 18 at the first time point t1 to the position B2 of the forceps 18 at the second time point t2. Here, the movement vector B1B2 is either predefined in the coordinate system of the endoscope 13, or is obtained by transforming vector information, which is obtained in another coordinate system on the side of the drive device 15, into the coordinate system of the endoscope 13.

The second identifying unit 52 identifies the second position and orientation information (vector CB2) at the second time point t2 based on the second position and orientation information (vector CB1) at the first time point t1 and the drive information (movement vector B1B2). A vector CB2 from the position C to the position B2 can be obtained by using the following equation according to a basic rule of vector calculation. $Vector CB 2 = vector CB 1 + movement vector B 1 B 2$

The estimation unit 54 estimates the first position and orientation information (vector CA2) at the second time point t2 based on the relative vector B1A1 and the second position and orientation information (vector CB2) at the second time point t2. A vector CA2 from the position C to the position A2 can be obtained by using the following equation according to a basic rule of vector calculation. $\begin{matrix}Vector CA 2 = vector CB 2 + vector B 2 A 2 \\ = vector CB 1 + movement vector B 1 B 2 + relative vector B 1 A 1\end{matrix}$

### Modification Example 2-2

In the above-described embodiment, a form has been described in which the relative positional relationship between the ultrasound probe 14 and the forceps 18 is fixed and does not change, but the present disclosure is not limited to this. For example, in a case where a rotation deviation and a position deviation that have occurred while the forceps 18 are connected to the ultrasound probe 14 are known, those deviations can be reflected in the relative positional relationship.

A method of estimating the first position and orientation information and a method of identifying the second position and orientation information in this case will be described with reference to FIG. 17. The acquisition unit 50 acquires variation information indicating an amount of variation in the relative positional relationship after the first time point t1. The term "variation information" refers to a value and/or vector information that quantitatively indicates a rotation deviation and a position deviation that have occurred while the forceps 18 are connected to the ultrasound probe 14. The variation information can be detected, for example, by a sensor attached to the robot arm or based on the surgical field image 21.

The derivation unit 53 corrects the relative positional relationship based on the variation information. That is, in a case where the relative vector B1A1 derived in advance at the first time point t1 deviates, the derivation unit 53 updates the relative vector B1A1 itself based on the deviation amount.

For example, an ideal position of the ultrasound probe 14 at the second time point t2, which is estimated based on the position C of the endoscope 13, the position B2 of the forceps 18 at the second time point t2, and the relative vector B1A1, is denoted by A2o. In addition, an actual position of the ultrasound probe 14 at the second time point t2 is denoted by A2. A variation vector from the ideal position A2o to the actual position A2 is denoted by A2oA2. A vector CA2 from the position C to the position A2 can be obtained by using the following equation according to a basic rule of vector calculation. $\begin{matrix}Vector CA 2 = vector CA 2 o + variation vector A 2 oA 2 \\ = vector CB 2 + vector B 2 A 2 + variation vector A 2 oA 2\end{matrix}$

### Third Embodiment

The medical support device 11 according to the present embodiment identifies the second position and orientation information based on an auxiliary image captured from a direction different from a direction of the surgical field image 21. As a result, the surgical field SF can be captured at an angle different from the viewpoint of the endoscope 13, thereby further improving general-purpose properties. In the following description, a part of the description overlapping with the first embodiment and/or the second embodiment will be omitted.

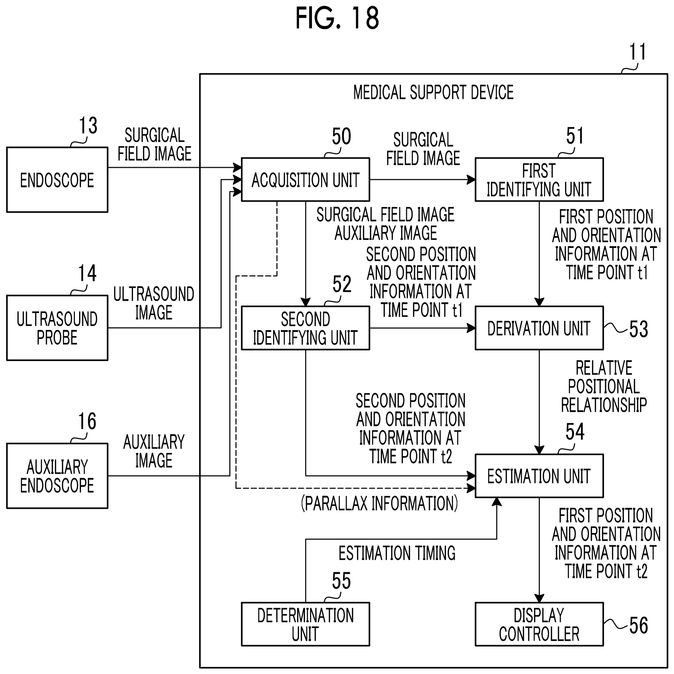

FIG. 18 is a block diagram showing an example of a functional configuration of the medical support device 11 according to the present embodiment. The medical support device 11 comprises an acquisition unit 50, a first identifying unit 51, a second identifying unit 52, a derivation unit 53, an estimation unit 54, a determination unit 55, and a display controller 56 as functional units. The processor 41 reads out the medical support program 44 from the storage 43 and executes the medical support program 44 on the RAM 42, so that the processor 41 functions as each functional unit.

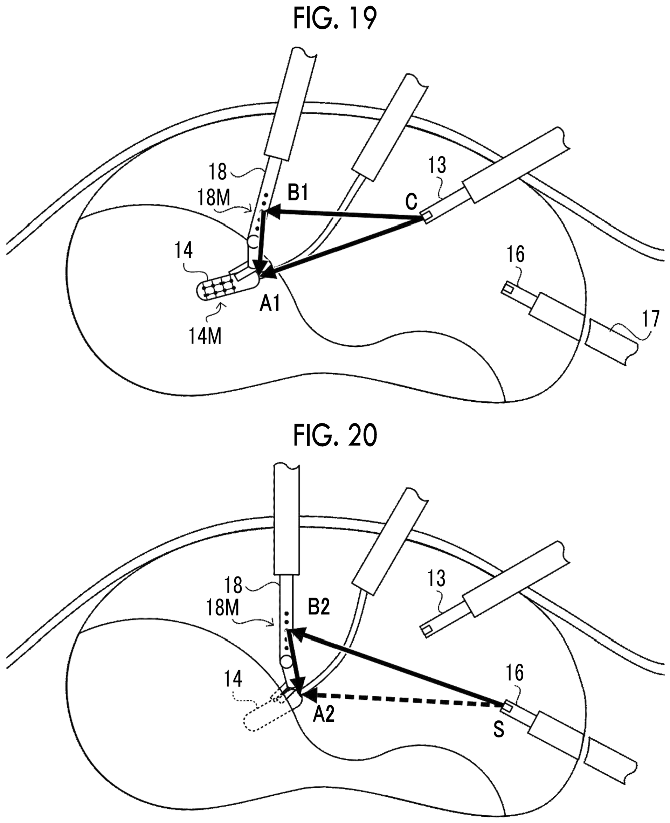

The acquisition unit 50 acquires an auxiliary image obtained by imaging the surgical field SF with a camera from a direction different from the direction of the surgical field image 21. For example, as shown in FIG. 19, an auxiliary endoscope 16 may be inserted into the body via a trocar 17 different from the trocar 17 for the endoscope 13, and the surgical field SF may be imaged by the auxiliary endoscope 16 to acquire the auxiliary image.

A method of estimating the first position and orientation information and a method of identifying the second position and orientation information in this case will be described with reference to FIGS. 19 and 20. FIG. 19 shows a state of the surgical field SF at the first time point t1. At the first time point t1, the first position and orientation information and the second position and orientation information can be identified from the surgical field image 21. Therefore, as in the first embodiment, the relative vector B1Al is obtained from the surgical field image 21 by using the following equation. $Vector B 1 A 1 = vector CA 1 - vector CB 1$

On the other hand, FIG. 20 shows a state of the surgical field SF at the second time point t2. In the present embodiment, it is assumed that it is difficult to identify the first position and orientation information and the second position and orientation information from the surgical field image 21 at the second time point t2. In this case, the second identifying unit 52 identifies the second position and orientation information at the first time point t1 and the second time point t2 based on the feature portion of the second medical instrument (forceps 18) included in the auxiliary image.

The estimation unit 54 estimates the first position and orientation information at the second time point t2 based on the relative positional relationship and the second position and orientation information at the second time point t2 identified from the auxiliary image. A position of the ultrasound probe 14 in the surgical field SF at the second time point t2 is denoted by A2, a position of the forceps 18 is denoted by B2, and a position of the auxiliary endoscope 16 (that is, a viewpoint of the auxiliary image) is denoted by S. A vector SA2 from the position S to the position A2 can be obtained by using the following equation according to a basic rule of vector calculation. $Vector SA 2 = vector SB 2 + relative vector B 1 A 1$

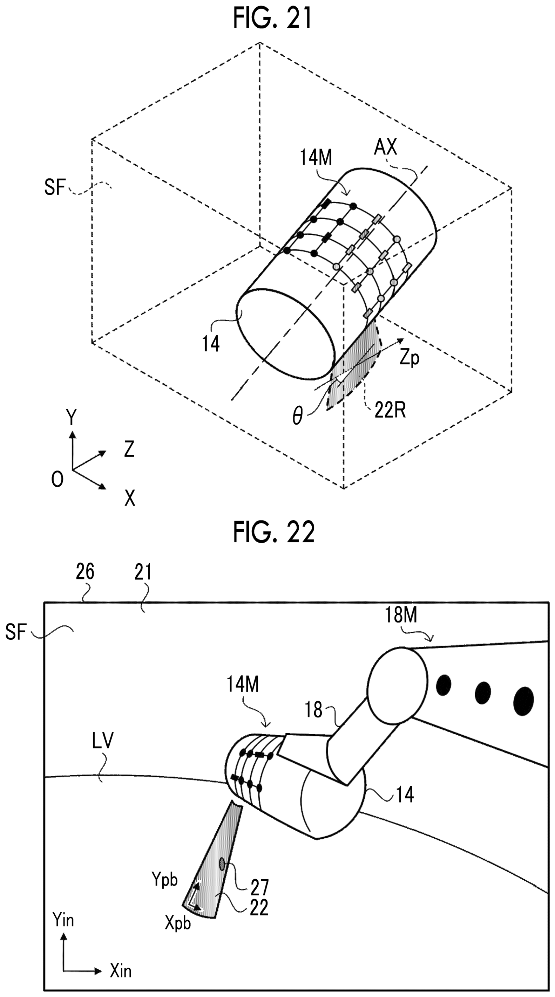

The display controller 56 may select the surgical field image 21 or the auxiliary image based on the first position and orientation information, and perform control of superimposing and displaying the ultrasound image 22 on the selected image. For example, as shown in FIGS. 21 and 22, the smaller an angle θ representing a direction of the ultrasound image 22, the more difficult it is to observe the ultrasound image 22 in the superimposed image 26. Therefore, the display controller 56 may select an image in which the angle θ representing the direction of the ultrasound image 22 is close to vertical, so that the ultrasound image 22 does not become excessively elongated. The method shown in FIG. 21 is the same as that of FIGS. 4 and 6, and the method shown in FIG. 22 is the same as that of FIGS. 5 and 7.

In addition, for example, the display controller 56 may select a camera closer to the ultrasound probe 14 so that the ultrasound image 22 is displayed larger. With such a form, the ultrasound image 22 in the superimposed image 26 does not become too small or excessively elongated, visibility can be improved, and the movement of the position and the orientation of the ultrasound probe 14 in the surgical field SF can be easily understood.

### Modification Example 3-1

In the above-described embodiment, the auxiliary image may be captured to track the second medical instrument (forceps 18). That is, the auxiliary endoscope 16 may be configured to receive mechanical or optical control such that the forceps 18 are always kept within an angle of view, preferably within the center of the angle of view. For example, the auxiliary endoscope 16 may be driven by the robot arm so that a blind spot portion that is not visible in the surgical field image 21 can be tracked and captured using the auxiliary image. This allows the forceps 18 to be always held by at least one of the endoscope 13 or the auxiliary endoscope 16, thereby further improving general-purpose properties.

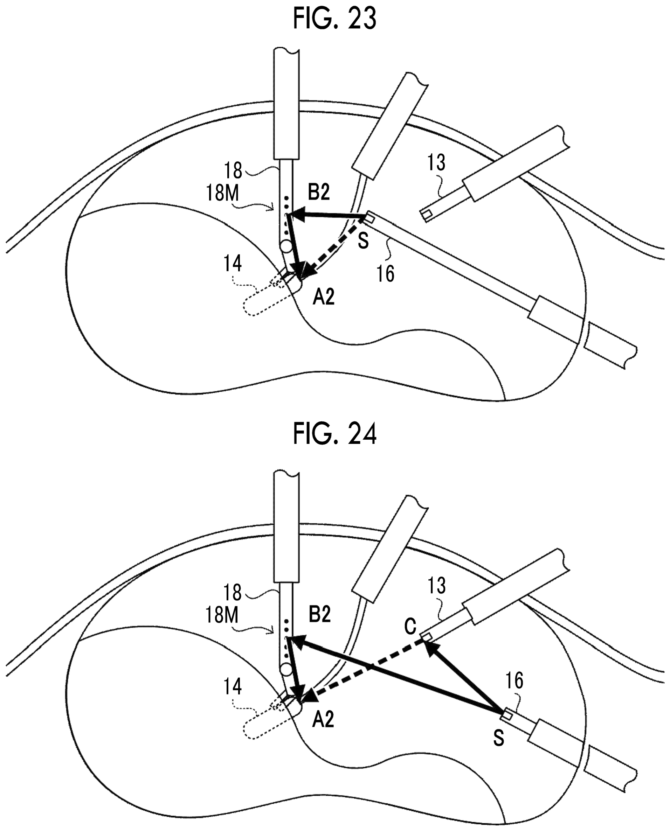

In addition, in this case, as shown in FIG. 23, the vector SA2 from the position S to the position A2 can be shortened as compared with a case where no tracking is performed as in FIG. 20. Therefore, errors can be reduced, and the accuracy of estimating the position and the orientation of the ultrasound probe 14 can be improved.

### Modification Example 3-2

In the above-described embodiment, the first position and orientation information and the second position and orientation information identified based on the auxiliary image are information based on a coordinate system within the auxiliary image. Therefore, for example, in a case where the first position and orientation information and the second position and orientation information are identified (estimated) based on each of the surgical field image 21 and the auxiliary image at each time point, the pieces of information cannot be treated on the same basis. Therefore, transformation for treating both the endoscope 13 and the auxiliary endoscope 16 on the same basis may be performed in consideration of the parallax between the endoscope 13 and the auxiliary endoscope 16.

Specifically, the acquisition unit 50 acquires parallax information of the surgical field image 21 and the auxiliary image. As shown in FIG. 24, the parallax information can be defined by, for example, a parallax vector SC from the position S of the auxiliary endoscope 16 to the position C of the endoscope 13.

The estimation unit 54 estimates the first position and orientation information (vector CA2) at the second time point t2 relative to the viewpoint C of the surgical field image 21 based on the relative positional relationship (relative vector B1A1), the second position and orientation information (vector SB2) at the second time point t2 identified based on the auxiliary image, and the parallax information (vector SC). A vector CA2 from the position C to the position A2 can be obtained by using the following equation according to a basic rule of vector calculation. $Vector CA 2 = vector SB 2 + relative vector B 1 A 1 - vector SC$

### Modification Example 3-3

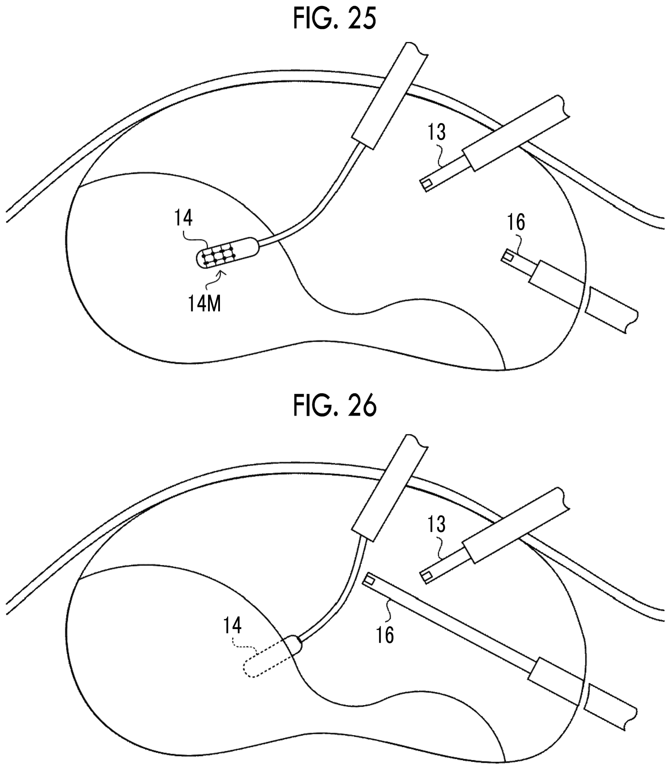

The form using the auxiliary image can also be applied to a surgery other than the robotic surgery. For example, as shown in FIG. 25, a form may be adopted in which the medical instrument such as the ultrasound probe 14 is imaged by a plurality of cameras (the endoscope 13 and the auxiliary endoscope 16) in a situation in which the forceps 18 are not inserted. This makes it easier to grasp the relative positional relationships between medical instruments and the spatial positions of the medical instruments with high accuracy, even in a complicated surgical environment such as inside a body cavity.

Further, as shown in FIG. 26, the auxiliary endoscope 16 may capture the auxiliary image to track the medical instrument such as the ultrasound probe 14.

In each of the above-described embodiments, a form has been described in which the position and the orientation of the ultrasound probe 14 in the surgical field SF are estimated using the marker 14M, but the present disclosure is not limited to this. For example, the position and the orientation may be estimated by detecting a characteristic shape of the ultrasound probe 14 from the surgical field image 21 through image analysis.

In each of the above-described embodiments, a form has been described in which the ultrasound probe 14 is used as an example of the medical instrument that is inserted into the body of the patient PT and images the internal structure of the target part, but the present disclosure is not limited to this. For example, a medical probe such as an optical coherence tomography (OCT) probe may be applied as such a medical instrument.

In each of the above-described embodiments, the body cavity such as the abdomen and the chest cavity has been described as an example of the surgical field SF, but the present disclosure is not limited to this. For example, as the surgical field SF, an upper gastrointestinal tract such as the esophagus, a lower gastrointestinal tract such as the intestine, or a tubular organ such as a bronchus may be used.

In each of the above-described embodiments, a form has been described in which the position and the orientation of the ultrasound probe 14 and the forceps 18 inserted into the body cavity are identified (estimated), but the present disclosure is not limited to this. For example, a form may be adopted in which the position and the orientation of an ultrasound probe that scans a body surface and various medical tools (for example, forceps, tweezers, a scalpel, a blade, a hook, and a needle holder) are identified (estimated). For example, by using a webcam as the camera, the technology of the present disclosure can also be applied to remote surgery via the body surface.

In the present embodiment, each process is executed by any computer. In addition, any computer may execute these processes using a processor as hardware, a program as software, or a combination thereof. In that case, the processor is configured to execute various processes in the present embodiment in cooperation with the program and can function as each unit or each means in the present embodiment. Further, the execution order of the process by the processor is not limited to the order described above and may be changed as appropriate. Any computer may be a general-purpose computer, a computer for a specific use, a workstation, or another system capable of executing each process.

The processor may be configured by one or more pieces of hardware, and the type of hardware is not limited. For example, the processor can be configured with a central processing unit (CPU), a micro processing unit (MPU), a programmable logic device such as a field programmable gate array (FPGA), a dedicated circuit for executing specific processing such as an application specific integrated circuit (ASIC), or hardware such as a graphic processing unit (GPU) or a neural processing unit (NPU). In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each process executed by the processor is not limited to the above order and may be changed as appropriate. The hardware is configured by an electric circuit (circuitry) in which circuit elements such as semiconductor elements are combined.

Further, the program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

In addition, in the above-described embodiment, the medical support program 44 has been described as being stored (installed) in the storage 43 in advance, but the present disclosure is not limited to this. The medical support program 44 may be provided in a form of being recorded on a recording medium, such as a compact disc read only memory (CD-ROM), a digital versatile disc read only memory (DVD-ROM), or a universal serial bus (USB) memory. In addition, the medical support program 44 may be downloaded from an external device via a network.

The technology of the present disclosure extends to any program products. The program product includes a product in any aspect for providing a program. For example, the program product includes a program provided through a network such as the Internet, and a non-transitory computer-readable recording media such as a CD-ROM, a DVD-ROM, and a USB memory in which the program is stored.

In the technology of the present disclosure, the embodiment and the modification examples described above can be combined as appropriate. The content of the above description and the content of the drawings are detailed explanations of the parts relating to the technology of the present disclosure, and are merely examples of the technology of the present disclosure. For example, description related to the above configurations, functions, actions, and effects is description related to an example of configurations, functions, actions, and effects of the parts according to the embodiments of the technology of the present disclosure. The scope of the invention is as defined by the claims.

The following appendices are disclosed with regard to the above embodiment.

### Supplementary Note 1

A medical support device comprising:
a processor,
in which the processor is configured to
   acquire, at a first time point, a surgical field image obtained by imaging, with a camera, a surgical field including a first medical instrument to which a marker is assigned,
   identify, based on the marker included in the surgical field image, first position and orientation information indicating a position and an orientation of the first medical instrument, the first position and orientation information being information at the first time point,
   acquire second position and orientation information indicating a position and an orientation of a second medical instrument connected to the first medical instrument, the second position and orientation information being information at the first time point and a second time point,
   derive a relative positional relationship between the first medical instrument and the second medical instrument based on the first position and orientation information and the second position and orientation information at the first time point, and
   estimate the first position and orientation information at the second time point based on the relative positional relationship and the second position and orientation information at the second time point.

### Supplementary Note 2

The medical support device according to Supplementary Note 1,
in which the surgical field image includes the second medical instrument to which a marker is assigned, and
the processor is configured to identify the second position and orientation information at at least one of the first time point or the second time point based on the marker included in the surgical field image.

### Supplementary Note 3

The medical support device according to Supplementary Note 1 or 2,
in which the surgical field image includes the second medical instrument, and
the processor is configured to identify the second position and orientation information at at least one of the first time point or the second time point based on a characteristic shape of the second medical instrument included in the surgical field image.

### Supplementary Note 4

The medical support device according to any one of Supplementary Notes 1 to 3,
in which the processor is configured to
acquire the second position and orientation information at the first time point,
acquire drive information of the second medical instrument, and
identify the second position and orientation information at the second time point based on the second position and orientation information at the first time point and the drive information.

### Supplementary Note 5

The medical support device according to Supplementary Note 4,
in which the processor is configured to
acquire the second position and orientation information at the first time point, the second position and orientation information being relative to a viewpoint of the surgical field image,
acquire drive information including a movement amount of the second medical instrument from the first time point to the second time point, and
identify the second position and orientation information at the second time point based on the second position and orientation information at the first time point and the drive information.

### Supplementary Note 6

The medical support device according to any one of Supplementary Notes 1 to 5,
in which the processor is configured to
acquire variation information indicating an amount of variation in the relative positional relationship after the first time point, and
correct the relative positional relationship based on the variation information.

### Supplementary Note 7

The medical support device according to any one of Supplementary Notes 1 to 6,
in which the processor is configured to
acquire an auxiliary image obtained by imaging the surgical field with a camera from a direction different from a direction in which the surgical field image is captured, and
identify the second position and orientation information at the first time point and the second time point based on a feature portion of the second medical instrument included in the auxiliary image.

### Supplementary Note 8

The medical support device according to Supplementary Note 7,
in which the auxiliary image is captured to track the second medical instrument.

### Supplementary Note 9

The medical support device according to Supplementary Note 7 or 8,
in which the processor is configured to
acquire parallax information of the surgical field image and the auxiliary image, and
estimate the first position and orientation information at the second time point relative to a viewpoint of the surgical field image based on the relative positional relationship, the second position and orientation information at the second time point identified based on the auxiliary image, and the parallax information.

### Supplementary Note 10

The medical support device according to any one of Supplementary Notes 7 to 9,
in which the first medical instrument is an ultrasound probe that captures an ultrasound image, and
the processor is configured to
   select the surgical field image or the auxiliary image based on the first position and orientation information, and
   perform control of superimposing and displaying the ultrasound image on the selected image.

### Supplementary Note 11

The medical support device according to any one of Supplementary Notes 1 to 10,
in which the processor is configured to
determine whether or not an amount of variation between the relative positional relationship at the first time point and the relative positional relationship at the second time point is within a predetermined threshold value, and
in a case where the amount of variation is within the threshold value, estimate the first position and orientation information at the second time point based on the relative positional relationship and the second position and orientation information at the second time point.

### Supplementary Note 12

The medical support device according to any one of Supplementary Notes 1 to 11,
in which the processor is configured to
perform a first determination of whether or not the second medical instrument is connected to the first medical instrument and a second determination of whether or not the marker and a feature portion of the second medical instrument are included in the surgical field image, and
set a time point at which both the first determination and the second determination are affirmative, as the first time point.

### Supplementary Note 13

The medical support device according to Supplementary Note 12,
in which the processor is configured to perform the first determination based on the surgical field image.

### Supplementary Note 14

The medical support device according to Supplementary Note 12 or 13,
in which the processor is configured to
acquire drive information of the second medical instrument, and
perform the first determination based on the drive information.

### Supplementary Note 15

The medical support device according to any one of Supplementary Notes 12 to 14,
in which the processor is configured to
perform the first determination and the second determination over time, and
in a case where the second determination is negative, set a time point at which both the immediately preceding first determination and second determination are affirmative, as the first time point.

### Supplementary Note 16

The medical support device according to any one of Supplementary Notes 1 to 15,
in which the processor is configured to, after the first time point, restrict a drive amount of the second medical instrument such that an amount of variation in the relative positional relationship over time is within a predetermined threshold value.

### Supplementary Note 17

The medical support device according to Supplementary Note 16,
in which the processor is configured to
determine whether or not the second medical instrument is connected to the first medical instrument, and
implement the restriction in a case where the determination is affirmative.

### Supplementary Note 18

The medical support device according to Supplementary Note 16 or 17,
in which the processor is configured to
receive designation as to whether or not to allow variation of the relative positional relationship after the first time point, and
implement the restriction in a case where it is designated that the variation of the relative positional relationship is not allowed.

### Supplementary Note 19

A medical support method executed by a computer, the medical support method comprising:
acquiring, at a first time point, a surgical field image obtained by imaging, with a camera, a surgical field including a first medical instrument to which a marker is assigned;
identifying, based on the marker included in the surgical field image, first position and orientation information indicating a position and an orientation of the first medical instrument, the first position and orientation information being information at the first time point;
acquiring second position and orientation information indicating a position and an orientation of a second medical instrument connected to the first medical instrument, the second position and orientation information being information at the first time point and a second time point;
deriving a relative positional relationship between the first medical instrument and the second medical instrument based on the first position and orientation information and the second position and orientation information at the first time point; and
estimating the first position and orientation information at the second time point based on the relative positional relationship and the second position and orientation information at the second time point.

### Supplementary Note 20

A medical support program for causing a computer to execute:
acquiring, at a first time point, a surgical field image obtained by imaging, with a camera, a surgical field including a first medical instrument to which a marker is assigned;
identifying, based on the marker included in the surgical field image, first position and orientation information indicating a position and an orientation of the first medical instrument, the first position and orientation information being information at the first time point;
acquiring second position and orientation information indicating a position and an orientation of a second medical instrument connected to the first medical instrument, the second position and orientation information being information at the first time point and a second time point;
deriving a relative positional relationship between the first medical instrument and the second medical instrument based on the first position and orientation information and the second position and orientation information at the first time point; and
estimating the first position and orientation information at the second time point based on the relative positional relationship and the second position and orientation information at the second time point.

## Claims

1. A medical support device (11) comprising a processor (41), wherein the processor (41) is configured to:
acquire, at a first time point (t1), a surgical field image (21) obtained by imaging, with a camera (13,16), a surgical field (SF) including a first medical instrument to which a marker is assigned;
identify, based on the marker included in the surgical field image (21), first position and orientation information indicating a position and an orientation of the first medical instrument, the first position and orientation information being information at the first time point (t1);
acquire second position and orientation information indicating a position and an orientation of a second medical instrument connected to the first medical instrument, the second position and orientation information being information at the first time point (t1) and a second time point (t2);
derive a relative positional relationship between the first medical instrument and the second medical instrument based on the first position and orientation information and the second position and orientation information at the first time point (t1); and
estimate the first position and orientation information at the second time point (t2) based on the relative positional relationship and the second position and orientation information at the second time point (t2).

2. The medical support device (11) according to claim 1, wherein:
the surgical field image (21) includes the second medical instrument to which a marker is assigned, and
the processor (41) is configured to identify the second position and orientation information at at least one of the first time point (t1) or the second time point (t2) based on the marker included in the surgical field image (21).

3. The medical support device according to claim 1 or 2, wherein:
the surgical field image (21) includes the second medical instrument, and
the processor (41) is configured to identify the second position and orientation information at at least one of the first time point or the second time point (t2) based on a characteristic shape of the second medical instrument included in the surgical field image (21).

4. The medical support device (11) according to any one of claims 1 to 3, wherein:
the processor (41) is configured to:
acquire the second position and orientation information at the first time point (t1);
acquire drive information of the second medical instrument; and
identify the second position and orientation information at the second time point (t2) based on the second position and orientation information at the first time point (t1) and the drive information, and
optionally, the processor (41) is configured to:
acquire the second position and orientation information at the first time point (t1), the second position and orientation information being relative to a viewpoint of the surgical field image (21);
acquire drive information including a movement amount of the second medical instrument from the first time point (t1) to the second time point (t2); and
identify the second position and orientation information at the second time point (t2) based on the second position and orientation information at the first time point (t1) and the drive information.

5. The medical support device (11) according to any one of claims 1 to 4, wherein the processor (41) is configured to:
acquire variation information indicating an amount of variation in the relative positional relationship after the first time point (t1); and
correct the relative positional relationship based on the variation information.

6. The medical support device (11) according to any one of claims 1 to 5, wherein:
the processor (41) is configured to:
acquire an auxiliary image obtained by imaging the surgical field (SF) with a camera (13,16) from a direction different from a direction in which the surgical field image (21) is captured; and
identify the second position and orientation information at the first time point (t1) and the second time point (t2) based on a feature portion of the second medical instrument included in the auxiliary image, and
optionally, the auxiliary image is captured to track the second medical instrument.

7. The medical support device (11) according to claim 6, wherein the processor (41) is configured to:
acquire parallax information of the surgical field image (21) and the auxiliary image; and
estimate the first position and orientation information at the second time point (t2) relative to a viewpoint of the surgical field image (21) based on the relative positional relationship, the second position and orientation information at the second time point (t2) identified based on the auxiliary image, and the parallax information.

8. The medical support device (11) according to claim 6 or 7, wherein:
the first medical instrument is an ultrasound probe (14) that captures an ultrasound image, and
the processor (41) is configured to:
select the surgical field image (21) or the auxiliary image based on the first position and orientation information; and
perform control of superimposing and displaying the ultrasound image on the selected image.

9. The medical support device (11) according to any one of claims 1 to 8, wherein the processor (41) is configured to:
determine whether or not an amount of variation between the relative positional relationship at the first time point (t1) and the relative positional relationship at the second time point (t2) is within a predetermined threshold value; and
in a case where the amount of variation is within the threshold value, estimate the first position and orientation information at the second time point (t2) based on the relative positional relationship and the second position and orientation information at the second time point (t2).

10. The medical support device (11) according to any one of claims 1 to 9, wherein:
the processor (41) is configured to:
perform a first determination of whether or not the second medical instrument is connected to the first medical instrument and a second determination of whether or not the marker and a feature portion of the second medical instrument are included in the surgical field image (21); and
set a time point at which both the first determination and the second determination are affirmative, as the first time point (t1), and
optionally, the processor is configured to perform the first determination based on the surgical field image (21); or
optionally, the processor (41) is configured to:
acquire drive information of the second medical instrument; and
perform the first determination based on the drive information.

11. The medical support device (11) according to claim 10, wherein the processor is configured to:
perform the first determination and the second determination over time; and
in a case where the second determination is negative, set a time point at which both the immediately preceding first determination and second determination are affirmative, as the first time point (t1).

12. The medical support device (11) according to any one of claims 1 to 11, wherein:
the processor (41) is configured to, after the first time point (t1), restrict a drive amount of the second medical instrument such that an amount of variation in the relative positional relationship over time is within a predetermined threshold value, and
optionally, the processor (41) is configured to:
determine whether or not the second medical instrument is connected to the first medical instrument; and
implement the restriction in a case where the determination is affirmative.

13. The medical support device (11) according to claim 12, wherein the processor (41) is configured to:
receive designation as to whether or not to allow variation of the relative positional relationship after the first time point (t1); and
implement the restriction in a case where it is designated that the variation of the relative positional relationship is not allowed.

14. A medical support method executed by a computer, the medical support method comprising:
acquiring, at a first time point (t1), a surgical field image (21) obtained by imaging, with a camera, a surgical field (SF) including a first medical instrument to which a marker is assigned;
identifying, based on the marker included in the surgical field image (21), first position and orientation information indicating a position and an orientation of the first medical instrument, the first position and orientation information being information at the first time point (t1);
acquiring second position and orientation information indicating a position and an orientation of a second medical instrument connected to the first medical instrument, the second position and orientation information being information at the first time point (t1) and a second time point (t2);
deriving a relative positional relationship between the first medical instrument and the second medical instrument based on the first position and orientation information and the second position and orientation information at the first time point (t1); and
estimating the first position and orientation information at the second time point (t2) based on the relative positional relationship and the second position and orientation information at the second time point (t2).

15. A computer-readable storage medium storing a medical support program for causing a computer to execute:
acquiring, at a first time point (t1), a surgical field image (21) obtained by imaging, with a camera, a surgical field (SF) including a first medical instrument to which a marker is assigned;
identifying, based on the marker included in the surgical field image (21), first position and orientation information indicating a position and an orientation of the first medical instrument, the first position and orientation information being information at the first time point (t1);
acquiring second position and orientation information indicating a position and an orientation of a second medical instrument connected to the first medical instrument, the second position and orientation information being information at the first time point (t1) and a second time point (t2);
deriving a relative positional relationship between the first medical instrument and the second medical instrument based on the first position and orientation information and the second position and orientation information at the first time point (t1); and
estimating the first position and orientation information at the second time point (t2) based on the relative positional relationship and the second position and orientation information at the second time point (t2).
